# EUROPEAN PATENT APPLICATION

(11) **EP 1 026 155 A1**
(43) Date of publication of application: **09.08.2000**
(21) Application number: 98943032.7
(22) Date of filing: 18.09.1998
(51) Int. Cl.: C07C 401/00, C07C 35/17, C07C 49/517, C07F 7/18, C07F 9/53

(54) **VITAMIN D DERIVATIVES AND PROCESS FOR PRODUCING THE SAME**

(30) Priority: 19.09.1997 JP 25547097
(71) Applicant: CHUGAI SEIYAKU KABUSHIKI KAISHA, Tokyo, 115-8543 (JP)
(72) Inventor: HATAKEYAMA, Susumi, Nagasaki-shi, Nagasaki 852-8054 (JP); IWABUCHI, Yoshiharu, Nagasaki-shi, Nagasaki 852-8156 (JP)
(74) Representative: VOSSIUS & PARTNER
(86) International application number: JP9804200
(87) International publication number: WO9915499

(57) **Abstract**

The object of the present invention is to provide a novel 22-oxavitamin D derivative having a hydroxy group at both 1α- and 3α-positions. According to the present invention, there is provided 22-oxavitamin D derivatives having the following general formula (I): 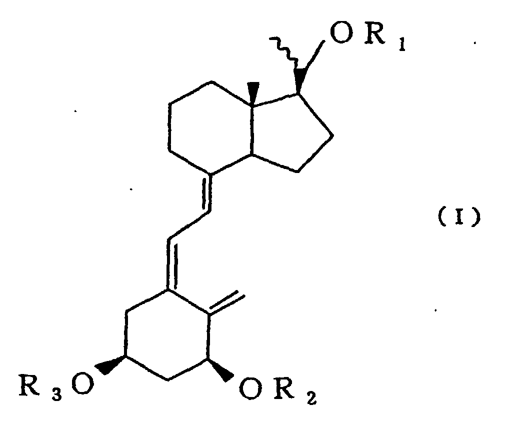 (wherein R₁ represents a straight or branched C₄-C₁₀ alkyl group having 1 to 3 hydroxy groups, and R₂ and R₃ each independently represents a hydrogen atom or a protecting group),
intermediates for the synthesis of the derivatives, and a method for producing them.

## Description

### TECHNICAL FIELD

The present invention relates to novel 3-epi-22-oxavitamin D derivatives, intermediates for synthesizing the derivatives, and a method for producing them. The 3-epi-22-oxavitamin D derivatives are expected to induce differentiation and inhibit proliferation and are expected to be useful as an antitumor agent, a therapeutic agent for rheumatic diseases and a therapeutic agent for psoriasis, and particularly as a therapeutic agent for hyperparathyroidism.

### BACKGROUND OF THE INVENTION

Recently, some of the physiological activities of vitamins D have been revealed. It is known that some of a certain vitamin D, for example, 1α,25-dihydroxy vitamin D₃, has a variety of physiological activities such as calcium metabolism-controlling activity, a proliferation-inhibiting activity and a differentiation-inducing activity on cells such as tumor cells, or an immune-controlling activity.

For example, Japanese Patent Publication (Kokai) No. 61-267550, Japanese Patent Publication (Kokai) No. 6-80626 and Japanese Patent Publication (Kokai) No. 6-256300 disclose 22-oxavitamin D derivatives each having a specific side chain and such compounds are described to have a proliferation-inhibiting activity and a differentiation-inducing activity.

However, the only known conventional known 22-oxavitamin D derivative that has been reported to date is one in which the hydroxy groups at 1- and 3-positions assume the trans configuration (i.e. 1α,3β-dihydroxy-22-oxavitamin D derivative); a derivative in which the hydroxy groups at 1- and 3-positions assume the cis configuration (i.e. 1α,3α-dihydroxy-22-oxavitamin D derivative) has not been reported yet.

A vitamin D derivative having a hydroxy group at both 1α-position and 3α-position is disclosed in J. Org. Chem. Vol. 58, No.7, 1993, p.1895-1899. However, this document does not disclose any 22-oxavitamin D derivatives.

### DISCLOSURE OF THE INVENTION

One object of the present invention is to provide a novel 22-oxavitamin D derivative having a hydroxy group at both 1α- and 3α-positions.

Another object of the present invention is to provide a novel intermediate useful in the synthesis of a 22-oxavitamin D derivative having a hydroxy group at both 1α-and 3α-positions.

Still another object of the present invention is to establish a synthesis route for the synthesis of a 22-oxavitamin D derivative having a hydroxy group at both 1α- and 3α-positions.

According to one aspect of the present invention, there is provided a 22-oxavitamin D derivative having the following general formula (I): 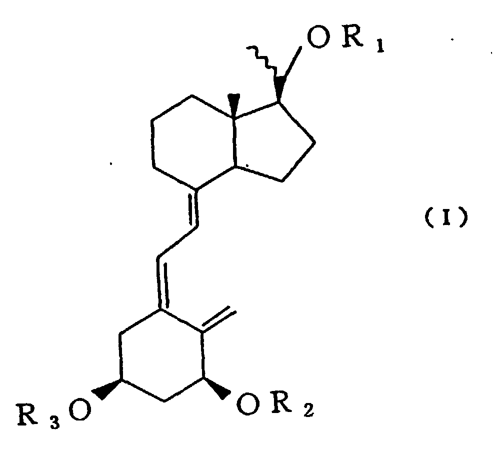 (wherein R₁ represents a straight or branched C₄-C₁₀ alkyl group having 1 to 3 hydroxy groups, and R₂ and R₃ each independently represents a hydrogen atom or a protecting group).

Preferably, R₁ represents a group having the following general formula (II): 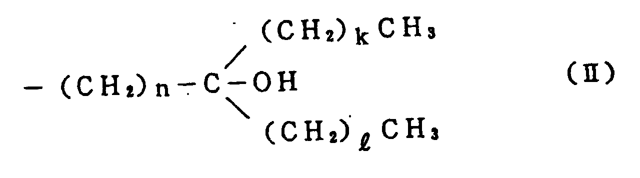 (wherein n represents an integer of 1 to 3 and k and l each independently represents an integer of 0 to 2).

Particularly preferably, R₁ represents 3-hydroxy-3-methyl-butyl group.

Although the vitamin D derivatives of the present invention can have either S or R configuration as the steric configuration at 20-position, S configuration is preferred.

According to a second aspect of the present invention, there is provided a compound having the general formula (III): 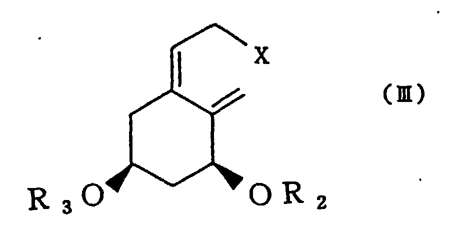 (wherein X represents a hydroxy group, a chlorine atom or a diphenylphosphine oxide group, and R₂ and R₃ each independently represents a hydrogen atom or a protecting group). The compound of the general formula (III) is novel and useful as an intermediate for synthesizing a 22-oxavitamin D derivative of the general formula (I).

According to a third aspect of the present invention, there is provided a compound having the following general formula (IV): 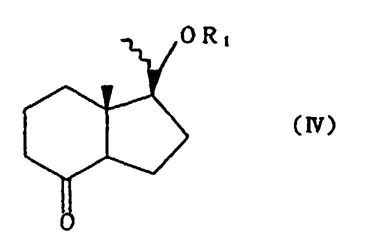 (wherein R₁ represents a straight or branched C₄-C₁₀ alkyl group having 1 to 3 hydroxy groups). The compound of the general formula (IV) is a novel compound and useful as an intermediate for synthesizing a 22-oxavitamin D derivative of the general formula (I).

According to a fourth aspect of the present invention, there is provided a method of producing a 22-oxavitamin D derivative having the following general formula (I): 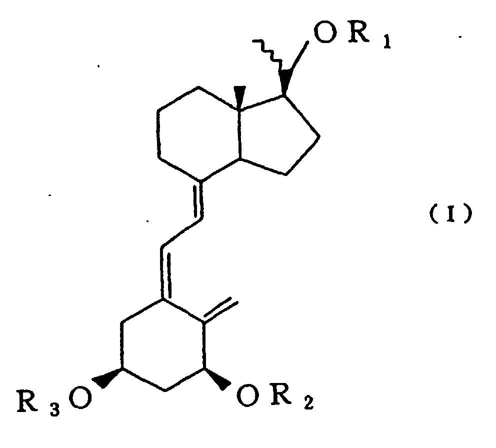 (wherein R₁ represents a straight or branched C₄-C₁₀ alkyl group having 1 to 3 hydroxy groups, and R₂ and R₃ each independently represents a hydrogen atom or a protecting group)
said method comprising reacting a compound having the following general formula (IIIa): 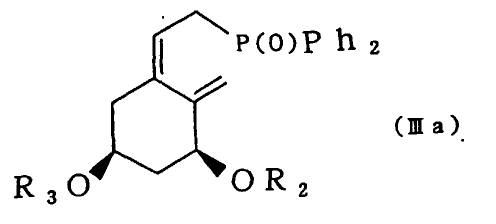 (wherein R₂ and R₃ each independently represents a hydrogen atom or a protecting group)
with a compound having the following general formula (IV): 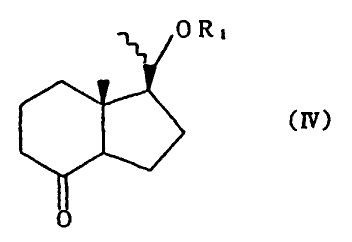 (wherein R₁ represents a straight or branched C₄-C₁₀ alkyl group having 1 to 3 hydroxy groups)
in a solvent in the presence of a base.

### BEST MODE FOR CARRYING OUT THE INVENTION

In the disclosure of the present invention, R₁ represents a straight or branched C₄-C₁₀ alkyl group having 1 to 3 hydroxy groups. Non-limiting examples of R₁ include a 3-hydroxy-3-methylbutyl group, a 2-hydroxy-3-methylbutyl group, a 4-hydroxy-3-methylbutyl group, a 2,3-dihydroxy-3-methylbutyl group, a 2,4-dihydroxy-3-methylbutyl group, a 3,4-dihydroxy-3-methylbutyl group, a 3-hydroxy-3-ethylpentyl group, a 2-hydroxy-3-ethylpentyl group, a 4-hydroxy-3-ethylpentyl group, a 2,3-dihydroxy-3-ethylpentyl group, a 2,4-dihydroxy-3-ethylpentyl group, a 3,4-dihydroxy-3-ethylpentyl group, a 4-hydroxy-4-methylpentyl group, a 3-hydroxy-4-methylpentyl group, a 5-hydroxy-4-methylpentyl group, a 3,4-dihydroxy-4-methylpentyl group, a 3,5-dihydroxy-4-methylpentyl group, a 4,5-dihydroxy-4-methylpentyl group, a 3-hydroxy-3-(n-propyl)hexyl group, a 4-hydroxy-3-(n-propyl)hexyl group, a 2-hydroxy-3-(n-propyl)hexyl group, a 2,3-dihydroxy-3-(n-propyl)hexyl group, a 3,4-dihydroxy-3-(n-propyl)hexyl group, a 2,4-dihydroxy-3-(n-propyl)hexyl group, a 3-hydroxy-4-ethylhexyl group, a 4-hydroxy-4-ethylhexyl group, a 5-hydroxy-4-ethylhexyl group, a 3,4-dihydroxy-4-ethylhexyl group, a 3,5-dihydroxy-4-ethylhexyl group, a 4,5-dihydroxy-4-ethylhexyl group, a 4-hydroxy-5-methylhexyl group, a 5-hydroxy-5-methylhexyl group, a 6-hydroxy-5-methylhexyl group, a 4,5-dihydroxy-5-methylhexyl group, a 4,6-dihydroxy-5-methylhexyl group, a 5,6-dihydroxy-5-methylhexyl group, a 5-hydroxy-6-methylheptyl group, a 6-hydroxy-6-methylheptyl group, a 7-hydroxy-6-methylheptyl group, a 5,6-dihydroxy-6-methylheptyl group, a 5,7-dihydroxy-6-methylheptyl group, a 6,7-dihydroxy-6-methylheptyl group, a 4-hydroxy-5-ethylheptyl group, a 5-hydroxy-5-ethylheptyl group, a 6-hydroxy-5-ethylheptyl group, a 4,5-dihydroxy-5-ethylheptyl group, a 4,6-dihydroxy-5-ethylheptyl group, a 5,6-dihydroxy-5-ethylheptyl group, a 3-hydroxy-4-(n-propyl)heptyl group, a 4-hydroxy-4-(n-propyl)heptyl group, a 5-hydroxy-4-(n-propyl) heptyl group, a 3,4-dihydroxy-4-(n-propyl)heptyl group, a 3,5-dihydroxy-4-(n-propyl)heptyl group and a 4,5-dihydroxy-4-(n-propyl)heptyl group.

In the disclosure of the present invention, R₂ and R₃ each represents a hydrogen atom or a protecting group. A protecting group means a group protecting a hydroxy group. Examples of such a protecting group are an acyl group, a substituted silyl group and a substituted or unsubstituted alkyl group, with an acyl group and a substituted silyl group being preferred.

Examples of the acyl group include an acetyl group, a benzoyl group, a substituted acetyl group and a substituted benzoyl group, as well as carbonate types and carbamate types, with an acetyl group being preferred. Examples of substituents on the acetyl group and the benzoyl group include a halogen atom, an alkyl group, an alkenyl group and an aryl group, with a fluorine atom, a chlorine atom, a methyl group, a phenyl group and an ethylidene group being preferred. Preferred examples of the substituted acetyl group include a chloroacetyl group, a trifluoroacetyl group, a pivaloyl group and a crotonoyl group. Preferred examples of the substituted benzoyl group include a p-phenylbenzoyl group and a 2,4,6-trimethylbenzoyl group.

Examples of the substituted silyl group include a trimethylsilyl group, a triethylsilyl group, a triisopropylsilyl group, a tert-butyldimethylsilyl group and a tert-butyldiphenylsilyl group, with a tert-butyldimethylsilyl group being preferred.

Examples of the substituted or unsubstituted alkyl group include a methyl group, a methoxymethyl group, a methylthiomethyl group, a tert-butylthiomethyl group, a benzyloxymethyl group, a p-methoxybenzyloxymethyl group, a 2-methoxyethoxymethyl group, a tetrahydropyranyl group, a tert-butyl group, an allyl group, a benzyl group, a p-methoxybenzyl group and an o- or p-nitrobenzyl group.

22-Oxavitamin D derivatives having the general formula (I) of the present invention can be produced by reacting a compound having the general formula (IIIa) with a compound having the general formula (IV) in a suitable solvent in the presence of a base.

Examples of the base used in the above reaction are alkyl lithiums such as n-butyl lithium, s-butyl lithium and phenyl lithium, lithium amides such as lithium diisopropylamide, lithium diethylamide and lithium tetramethylpiperazine, and lithium hexamethyldisilazane.

Examples of the solvent used for the above reaction are inert organic solvents, in particular ether solvents such as THF, diethyl ether, diisopropyl ether, 1,4-dioxane and diglyme (diethylene glycol dimethyl ether).

The reaction temperature is generally in the range of from -100C° to 0C°, preferably -80C° to -60C°.

The reaction time may be appropriately controlled, generally in the range of from 1 hour to 24 hours, preferably 3 hours to 9 hours.

Both the compounds having the general formula (III) and the compounds having the general formula (IV), which can be used as intermediates in the synthesis of 22-oxavitamin D derivatives having the general formula (I) of the present invention, are novel compounds and constitute another aspect of the present invention.

The compounds having the general formula (III) constitute the A ring structure of vitamin D derivatives and can be synthesized, for example, by the synthesis route described in the following Examples. The synthesis route indicated in Examples is shown below:

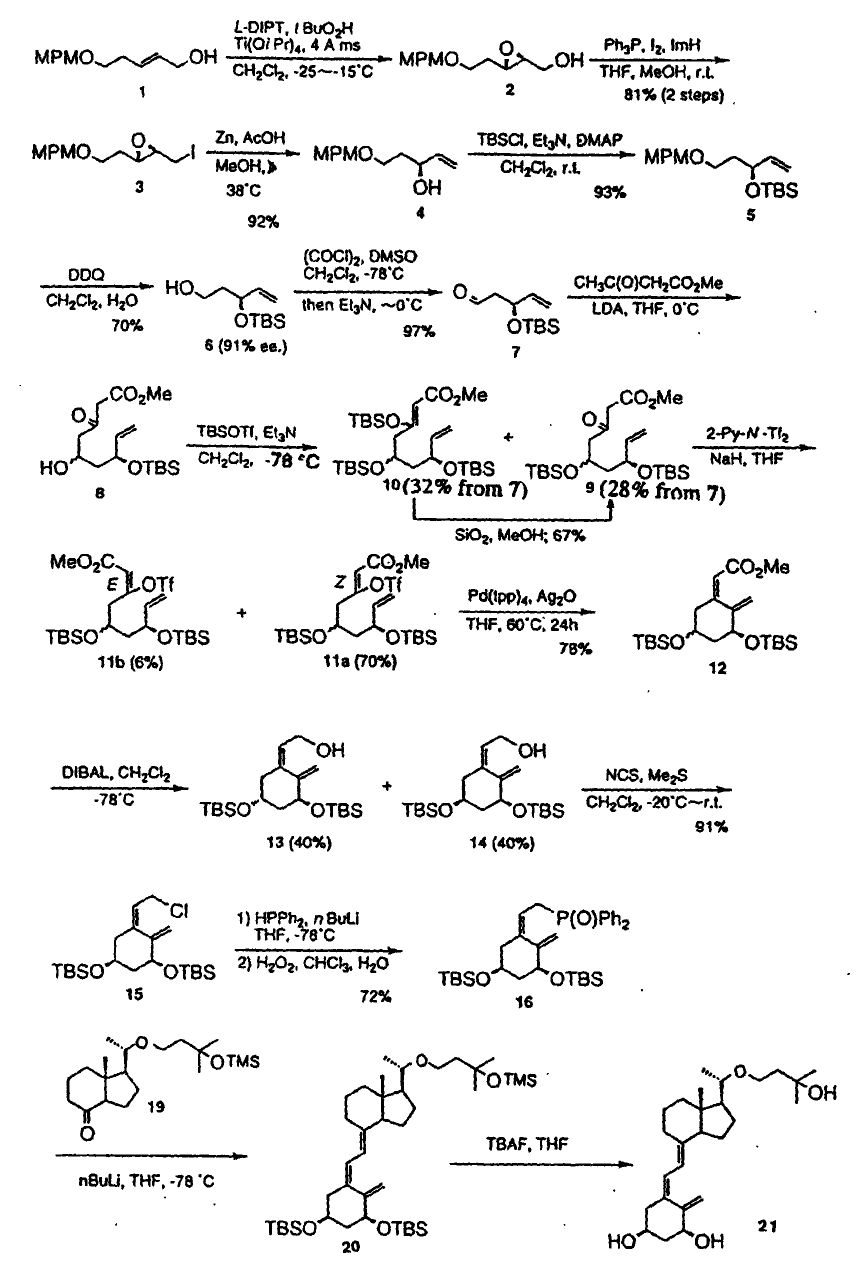

In the above reaction route, up to the stage of obtaining Compound 12 each compound is obtained as a mixture of epimers interms of the steric configuration of the substituent at a position corresponding to 3-position of the vitamin D derivative. The epimer mixture as Compound 12 is then converted to a mixture of Compound 13 and Compound 14 by a reducing agent such as i-Bu₂AlH, and these Compounds 13 and 14 can be separated by the usual isolating and purifying means such as column chromatography. Sterically-selective synthesis of the final end product of the present invention, 1α,3α-dihydroxy-22-oxavitamin D derivatives, is achieved by separating Compound 13 and Compound 14 from the stereoisomeric mixture of Compounds 13 and 14.

The synthesis route for obtaining Compound 12 is not particularly limited. Compound 12 is a known compound and can be obtained by, for example, the method described in J. Org. Chem., 1993, 58, 2523-2529.

The compounds having the general formula (IV) constitute the CD ring structure of vitamin D derivatives and can be synthesized, for example, using known final vitamin D derivatives as starting materials, as is described in Japanese Patent Publication (Kokai) No. 61-267550, Japanese Patent Publication (Kokai) No. 6-72994, Japanese Patent Publication (Kokai) No. 6-256300, Japanese Patent Publication (Kohyo) No. 4-503669, Japanese Patent Publication (Kohyo) No. 4-504573. In other words, a final vitamin D derivative is subjected to ozonolysis after protecting the hydroxy group thereof by a protecting group, and then subjected to reduction with NaBH₄ to give an alcohol compound. A ketonic compound of the general formula (IV) is obtained by oxidizing the alcohol compound with an appropriate oxidizing agent (e.g. n-Pr₄NRuO₄).

The whole content of Japanese Patent Application No. 9-255470, on which the claims for the priority of the present application is based, is incorporated herein by reference.

### Examples

The present invention will now be illustrated in detail with reference to the following Examples, however, it should be understood that the present invention is by no means limited thereto.

Unless otherwise indicated, commercially available reagents were used as such. By distillation, dry CH₂Cl₂ was prepared from CaH₂, and diethyl ether and tetrahydrofuran (THF) from sodium benzophenone ketyl. Kieselgel 60 (70-230 mesh, 230-240 mesh) manufactured by Merck was employed as silica gel for column chromatography. Kieselgel 60F₂₅₄ plates (0.25 mm and 0.5 mm) manufactured by Merck were employed for thin layer chromatography (TLC). Ultraviolet radiation (254 nm), iodine, a 2% anisaldehyde solution in ethanol acidified by sulfuric acid, a 5% phosphomolybdic acid solution in ethanol and a 2,4-dinitorophenyl hydrazine coloring reagent were used for coloring. Unity plus 500 and Gemini 300 and 200 manufactured by Varian were used for determining nuclear magnetic resonance spectra with tetramethylsilane or chloroform as an internal standard substance. FT/IR230 manufactured by JASCO Corporation was used for determining infrared absorption spectra. JMS-Dx303 manufactured by JEOL Ltd. was used for mass spectrometry and DIP370 manufactured by JASCO Corporation was used for determining optical rotation.

### Example 1: Preparation of (2S,3S)-2,3-epoxy-5-(4-methoxybenzyl)oxy-1-pentanal (Compound 2)

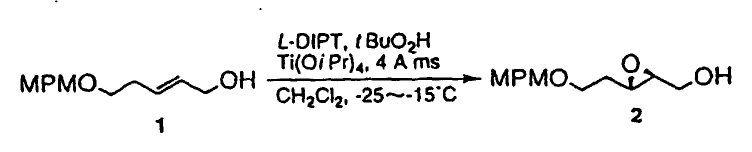

Under argon atmosphere at -23°C, Ti(O-i-Pr)₄ (468 µl, 1.58 millimoles (mmol)) was added to a solution of L-(+)-diisopropyl tartrate (474.0 mg, 2.03 mmol) and 4A molecular sieves (405 mg) in CH₂Cl₂ (12 ml) and the mixture was stirred for 10 min. After adding dropwise a solution of 5-(4-methoxybenzyl)oxy-2-pentene-1-ol (Compound 1) (5.00 g, 22.5 mmol) in CH₂Cl₂ (11 ml) and tert-butyl peroxide (2.97M solution in CH₂Cl₂; 15 ml, 45.0 mmol) thereto, the mixture was allowed to warm to -15°C and stirred for 2.5 days. The reaction mixture was mixed with a saturated aqueous Na₂SO₄ solution and diethyl ether, stirred for 2 hours at room temperature, filtered with Celite, concentrated and purified by silica gel column chromatography (SiO₂ = 250 g. hexane:ethyl acetate = 3:2) to give the titled Compound 2 (4.92 g, 20.7 mmol, 92%) as a colorless oil.
¹H NMR (300 MHz, CDCl₃) 1.70-1.99 (m, 3H), 2.97 (dt, J=2.7, 4.2 Hz, 1H), 3.10 (ddd, J=2.7, 5.1, 6.6 Hz, 1H), 3.58 (t, J=5.7 Hz, 2H), 3.62 (ddd, J=4.2, 7.2, 12.3 Hz, 1H), 3.81 (s, 3H), 3.90 (ddd, J=2.7, 5.7, 12.3 Hz, 1H), 4.45 (s, 2H), 6.88 (d, J=8.7Hz, 2H), 7.26 (d, J=8.7 Hz, 2H);
¹³C NMR (75 MHz, CDCl₃) 32.0, 53.7. 55.2, 58.5, 61.8, 66.5, 72.7, 113.8, 129.2, 130.2, 159.1;
FT-IR (neat) 3426, 1512, 1462, 1248, 1176, 1097, 819 cm⁻¹;
MS(EI) m/z 83 (100), 121, 137, 207, 238 (M⁺);
[α]_{D}¹⁸ -27.7°(c 0.90, CHCl₃);
HRMS (EI) C₁₃H₁₈O₄ Calcd. 238.1205; Found 238.1222;
TLC (hexane : ethyl acetate = 1:2, Rf0.5)

### Example 2: Preparation of (2S,3R)-2,3-epoxy-1-iodo-5-(4-methoxybenzyl) oxypentane (Compound 3)

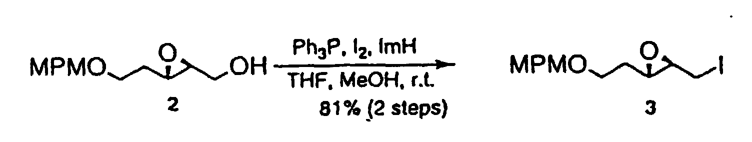

The (2S,3S)-2,3-epoxy-5-(4-methoxybenzyl)oxy-1-pentanol (4.00 g, 16.8 mmol) obtained in Example 1 was dissolved in THF-MeCN (4:1, 300 ml). To the solution, imidazole (8.58 g, 126 mmol), triphenylphosphine (13.2 g, 50.4 mmol) and iodine (12.8 g, 50.4 mmol) were added, followed by stirring for 30 min. at room temperature. The resulting reaction mixture was diluted by the addition of diethyl ether and then washed with a saturated aqueous NaHCO₃ solution, a saturated aqueous NaHSO₃ solution and a saturated aqueous sodium chloride. The organic layer was dried over MgSO₄, filtered, concentrated and then purified by silica gel column chromatography (SiO₂ = 250 g, hexane:ethyl acetate = 20:1) to give the titled Compound 3 (5.88 g, 16.9 mmol, 100%) as a pale yellow oil.
¹H NMR (300 MHz, CDCl₃) 1.73-1.95 (m, 2H), 2.98 (ddd, J=1.8, 5.1, 6.3 Hz, 1H), 3.06 (ddd, J=1.8, 6.6, 8.7 Hz, 1H), 3.07 (dd, J=6.3, 12.6 Hz, 1H), 3.22 (dd, J=8.7, 12.6 Hz, 1H), 3.57 (t, J=5.7 Hz, 2H). 3.81 (s, 3H). 6.89 (d, J=9.0 Hz, 2H), 7.27 (d, J=9.0 Hz, 2H);
¹³C NMR (75 MHz, CDCl₃) 5.0, 32.2, 55.3, 58.3, 60.3, 66.4, 72.8, 113.8, 129.3, 130.2, 159.2;
FT-IR (neat) 2948, 2859, 1612, 1512, 1460, 1361, 1301, 1249, 1174, 1100, 1033, 889, 1033, 820 cm⁻¹;
MS(EI) m/z 121 (100), 221, 348 (M⁺);
[α]D¹⁹ +4.3°(c 1.04, CHCL₃);
HRMS (EI) C₁₃H₁₇O₃I Calcd. 348.0222; Found 348.0216;
TLC (hexane : ethyl acetate = 5:1, Rf0.5)

### Example 3: Preparation of (3S)-5-(4-methoxybenzyl)oxy-1-penten-3-ol (Compound 4)

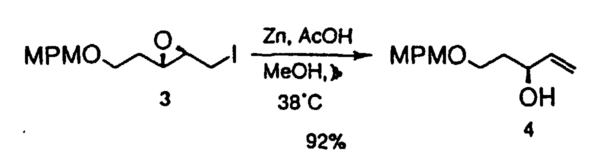

The (2S,3R)-2,3-epoxy-1-iodo-5-(4-methoxybenzyl)oxypentane (5.39 g, 15.5 mmol) obtained in Example 2 was dissolved in methanol (200 ml). To the solution, an activated zinc powder (3.04 g, 46.5 mmol) and acetic acid (8 ml) were added and subjected to reaction at 37°C for 1 hour under ultrasonic radiation. The resulting reaction mixture was diluted by the addition of diethyl ether and filtered with Celite to remove precipitates; the filtrate was washed with 5% hydrochloric acid, a saturated aqueous NaHCO₃ solution and a saturated aqueous sodium chloride. The organic layer was dried over MgSO₄, filtered, concentrated and then purified by silica gel column chromatography (SiO₂ = 90 g, henna: ethyl acetate = 7:2) to give the titled Compound 4 (3.18 g, 14.3 mmol, 92%) as a colorless oil.
¹H NMR (300 MHz, CDCl₃) 1.73-1.92 (m, 2H), 2.98 (d, J= 3.6 Hz, 1H), 3.56-3.73 (m, 2H), 3.81 (s, 3H), 4.28-4.38 (m, 1H), 4.45 (s, 2H), 5.10 (dt, J=1.2, 10.8 Hz, 1H), 5.27 (dt, J=1.2, 17.4 Hz, 1H), 5.87 (ddd, J=5.4, 10.8, 17.4 Hz, 1H), 6.88 (d, J=8.7 Hz, 2H), 7.25 (d, J=8.4 Hz, 2H);
¹³C NMR (75 MHz, CDCl₃) 36.1, 55.4, 68.1, 72.3, 73.1, 113.9, 114.7, 129.5, 129.8, 140.3, 159.3;
FT-IR (neat) 3427, 1612, 1513, 1461, 1363, 1249, 1095, 1033, 821 cm⁻¹;
MS(EI) m/z 121 (100), 222 (M⁺);
[α]_{D}²⁰ +8.5°(c 0.98, CHCl₃);
HRMS (EI) C₁₃H₁₈O₃ Calcd. 222.1256; Found 222.1249;
TLC (hexane : ethyl acetate = 2:1, Rf0.5)

### Example 4: Preparation of (3S)-3-(tert-butyldimethylsilyl)oxy-5-(4-methoxybenzyl)oxy-1-pentene (Compound 5)

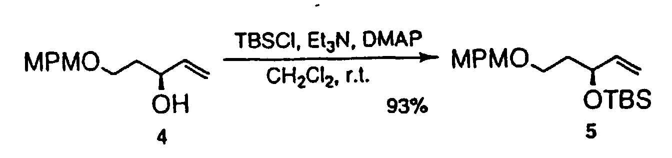

Under argon atmosphere at 0°C, triethylamine (5.0 ml, 35.9 mmol), 4-dimethylaminopyridine (169.0 mg, 1.38 mmol) and tert-butyldimethylsilyl chloride (97%; 5.15 g, 33.1 mmol) were added to a CH₂Cl₂ (100 ml) solution of the (3S)-5-(4-methoxybenzyl)oxy-1-penten-3-ol (5.15 g, 33.1 mmol) obtained in Example 3; the resulting mixture was allowed to warm to room temperature and stirred for 16 hours. The reaction mixture was mixed with a saturated NaHCO₃ solution and extracted with ethyl acetate; the extract was dried over MgSO₄, filtered, concentrated and then purified by flash silica gel column chromatography (SiO₂ = 120 g, hexane:ethyl acetate = 10:1) to give the titled Compound 5 (4.33 g, 12.9 mmol, 93%) as a colorless oil.
¹H NMR (300 MHz, CDCl₃) 0.03 (s, 3H), 0.05 (s, 3H), 0.89 (s, 9H), 1.77 (q, J= 7.7 Hz, 2H), 3.43-3.60 (m, 2H), 3.81 (s, 3H), 4.28 (q, J=6.6 Hz, 1H), 4.38 (d, J=11.0 Hz, 1H), 4.45 (d, J=11.0 Hz, 1H), 5.01 (dt, J=2.0, 10.0 Hz, 1H), 5.14 (dt, J=2.0, 17.0 Hz, 1H), 5.80 (ddd, J=6.6. 10.0, 17.0 Hz, 1H), 6.88 (d, J=8.7 Hz, 2H), 7.26 (d, J=8.7 Hz, 2H);
¹³C NMR (75 MHz, CDCl₃) -4.8. -4.3, 18.3, 26.0, 38.2, 55.4, 66.5, 70.9, 72.7, 113.7, 113.8, 129.4, 130.7, 141.7, 159.2;
FT-IR (neat) 1620, 1515, 1405, 1360, 1300, 1250, 1090, 1040, 920, 840 cm⁻¹;
MS(EI) m/z 122 (100), 279, 336 (M⁺);
[α]_{D}¹⁸ +2.0°(c 0.99, CHCl₃);
HRMS (EI) C₁₉H₃₂O₃Si Calcd. 336.2121; Found 336.2066;
TLC (hexane : ethyl acetate = 10:1, Rf0.5)

### Example 5: Preparation of (3S)-3-(tert-butyldimethylsilyl)oxy-4-penten-1-ol (Compound 6)

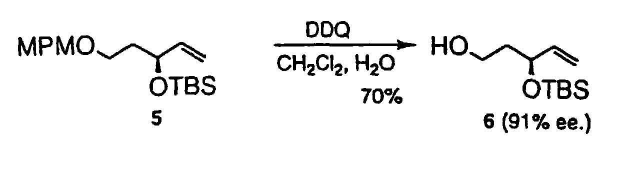

The (3S)-3-(tert-butyldimethylsilyl)oxy-5-(4-methoxybenzyl)oxy-1-pentene (531.4 mg. 1.58 mmol) obtained in Example 4 was dissolved in CH₂Cl₂ hydrate (5 ml). To the solution, DDQ (539.2 mg, 2.38 mmol) was added, followed by stirring for 15 min. The reaction mixture was mixed with an aqueous saturated NaHCO₃ solution (5 ml) and extracted with CH₂Cl₂ (25 ml x 3). The extracts were washed with a saturated aqueous NaHCO₃ solution (20 ml), dried over MgSO₄, filtered, concentrated and then purified by silica gel column chromatography (SiO₂ = 30 g, hexane:ethyl acetate = 10:1) to give the titled Compound 6 (377.2 mg, 1.89 mmol, quant.) as a pale yellow oil.
¹H NMR (300 MHz, CDCl₃) 0.06 (s, 3H), 0.10 (s, 3H), 0.91 (s, 9H), 1.65-1.78 (m, 1H), 1.79-1.91 (m, 1H), 2.46 (br t, J= 5.4 Hz, 1H), 3.66-3.76 (m, 1H), 3.78-3.88 (m, 1H), 4.42 (br q, J=6.0 Hz, 1H), 5.11 (dt, J=1.8. 9.3 Hz, 1H), 5.23 (dt, J=1.8, 17.4 Hz, 1H), 5.85 (ddd, J=5.7, 9.3, 17.4 Hz, 1H);
¹³C NMR (75 MHz, CDCl₃) -5.0, -4.3, 18.2, 25.9, 39.2, 60.2, 73.3, 114.5, 140.7;
FT-IR (neat) 3350, 1643, 1467, 1362, 1254, 1087, 1024, 922, 838 cm⁻¹;
MS(EI) m/z 75 (100), 159, 160, 171, 217 (M⁺);
[α]_{D}²⁰ +3.3°(c 0.94, CHCl₃);
HRMS (EI) C₉H₁₉OSi Calcd. 171.1205; Found 171.1204;
TLC (hexane : ethyl acetate = 4:1, Rf0.5)

### Example 6: Preparation of (3S)-3-(tert-butyldimethylsilyl)oxy-4-pentenal (Compound 7)

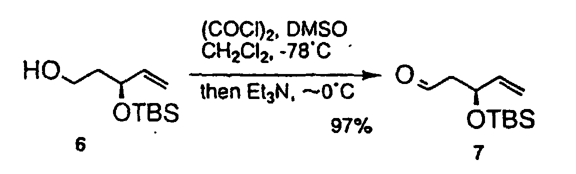

Oxalyl chloride (0.9 ml, 19.32 mmol) was stirred at -63°C for 30 min under argon atmosphere. The (3S)-3-(tert-butyldimethylsilyl)oxy-4-pentene-1-ol (1.114 g, 5.16 mmol) obtained in Example 5 was dissolved in CH₂Cl₂ (5 ml) and the solution was added dropwise to the oxalyl chloride. After stirring for 40 min., the reaction mixture was mixed with triethylamine (5.75 ml, 41.25 mmol) and the reaction temperature was gradually raised to room temperature. The reaction mixture was mixed with 0.1M HCl and diluted with ethyl acetate; the organic layer was washed with water and saturated aqueous sodium chloride, dried over MgSO₄, filtered and concentrated to give the titled Compound 7 (1.077 g. 5.03 mmol, 97%) as a pale yellow oil. This compound was used to the next reaction without further purification.
¹H NMR (300 MHz, CDCl₃) 0.06 (s, 3H), 0.07 (s, 3H), 0.88 (s, 9H), 2.5 (ddd, J=2.1, 4.8, 15.6 Hz, 1H), 2.62 (ddd, J=2.7, 6.6. 15.6 Hz, 1H), 4.65 (br q, J=5.1 Hz, 1H), 5.13 (dt, J=1.5, 10.5 Hz, 1H), 5.27 (dt, J=1.5, 17.1 Hz, 1H), 5.88 (ddd, J=5.7, 10.5, 17.1 Hz, 1H), 9.79 (br t, J=2.5 Hz, 1H);
MS(EI) m/z 75 (100), 157, 171, 185 (N-CHO);
TLC (hexane : ethyl acetate = 2:1, Rf0.5)

### Example 7: Preparation of methyl(5R and S, 7S)-7-(tert-butyldimethylsilyl)oxy-5-hydroxy-3-oxo-8-nonanoate (Compound 8)

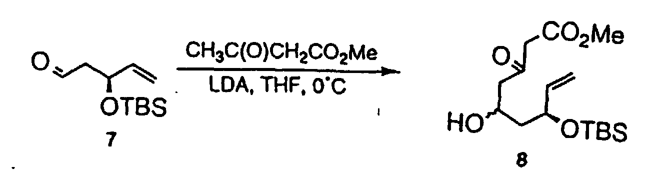

Under argon atmosphere, methyl acetoacetate (1.4 ml, 13 mmol) was added to a solution of lithium diisopropylamide in THF (0.7M; 28.7 mmol, 41 ml) as it was cooled at -78°C. followed by stirring at that temperature for 1 hour. Thereafter a solution of (3S)-3-(tert-butyldimethylsilyl)oxy-4-pentenal (1.077 g, 5.03 mmol) obtained in Example 6 in THF (5 ml) was added dropwise thereto, followed by further stirring for 1.5 hours. The reaction mixture was mixed with aqueous ammonia and extracted with ethyl acetate; the extract was washed with aqueous ammonia, dried over MgSO₄, filtered and concentrated to give a pale yellow oil (1.762 g). The obtained oil was used in the next reaction without further purification, however, a portion of the oil was purified by preparative thin layer silica gel chromatography (hexane:ethyl acetate = 5:2) to give the titled Compound 8.
¹H NMR (300 MHz, CDCl₃) 0.06 (s, 3H), 0.010 (s, 3H), 0.90 (s, 4.5H), 0.91 (s, 4.5H), 1.52-1.79 (m, 2H), 2.58-2.78 (m, 2H), 3.51 (s, 1H), 3.52 (s, 1H), 3.74 (s, 3H), 4.20-4.28 (m, 0.5H), 4.38 (br quint., J=6.6 Hz, 1H), 4.46-4.56 (m, 0.5H), [5.08 (br d, J=10.5 Hz), 5.12 (br d, J=12.0 Hz), 5.18 (br d, J-17.1 Hz), 5.25 (br d, J=17.1 Hz), 2H], 5.74-5.92 (m, 1H)

### Example 8: Preparation of methyl(5R and S, 7S)-5,7-di(tert-butyldimethylsilyl)oxy-3-oxo-8-nonanoate (Compound 9)

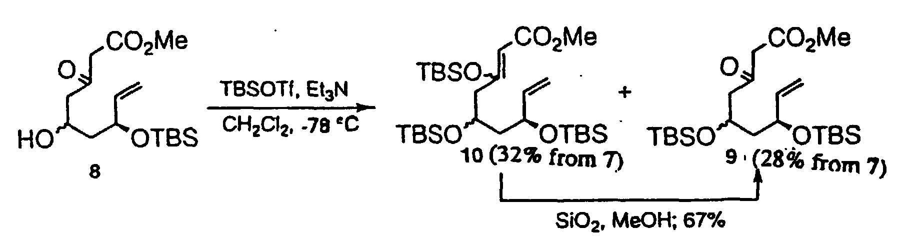

The crude product (1.7 g) containing methyl(5R and S, 7S)-7-(tert-butyldimethylsilyl)oxy-5-hydroxy-3-oxo-8-nonanoate as obtained in Example 7 was dissolved in CH₂Cl₂ (50 ml). To the solution, triethylamine (1.1 ml, 7.89 mmol) and tert-butyl dimethylsilyltriflate (1.42 ml, 6.18 mmol) were added under argon atmosphere at -78°C, followed by stirring for 20 min. The reaction mixture was alkalified by the addition of saturated aqueous ammonia and extracted with ethyl acetate. The extract was washed with saturated aqueous sodium chloride, dried over MgSO₄, filtered, concentrated and purified by column chromatography (SiO₂ = 100 g, hexane:ethyl acetate = 15:1) to give the titled Compound 9 (626 mg, 1.41 mmol, 28% yield from Compound 7) as a colorless oil and methyl(5R and S,7S)-3,5,7-tri(tert-butyldimethylsilyl)oxynona-2,8-dienoate (Compound 10) (902 mg, 1.62 mmol, 32% yield from Compound 7) as a colorless oil.

To a solution of Compound 10 (586.4 mg, 1.05 mmol) in methanol (58 ml), silica gel (5.90 g) was added, followed by stirring for 5 days. The reaction mixture was diluted with ethyl acetate, filtered through a cotton plug for removing silica gel and concentrated. The residue was purified by silica gel chromatography (SiO₂ = 15 g, hexane:ethyl acetate = 30:1) to give a colorless oil of the titled Compound 9 (313.7 mg, 0.707 mmol, 67%) as a mixture of C-5 epimers (1:1).
¹H NMR (300 MHz, CDCl₃) 0.00-0.10 (m, 12H), [0.86 (s), 0.87 (s), 0.88 (s), 0.89 (s), 18H], 0.57-1.82 (m, 2H), [2.69 (d, J=3.9 Hz), 2.70 (d, J=6.0 Hz), 2.73 (d, J=3.9 Hz), 2H], [3.47 (s), 3.48 (s), 2H], 4.07-4.35 (m, 2H), [5.04 (br d, J=16.5 Hz), 5.05 (br d, J=10.2 Hz), 1H], [5.14 (br d, J=17.4 Hz), 5.16 (bd d, J=15.9 Hz), 1H], 5.72-5.87 (m, 1H)

### Example 9: Preparation of methyl(Z,5R and S,7S)-5,7-bis(tert-butyldimethylsilyl)oxy-3-(trifluoromethanesulfonyl)oxynona-2,8-dienoate (Compound 11a) and methyl(E,5R and S,7S)-5,7-bis(tert-butyldimethylsilyl)oxy-3-(trifluoromethanesulfonyl)oxynona-2,8-dienoate (Compound 11b)

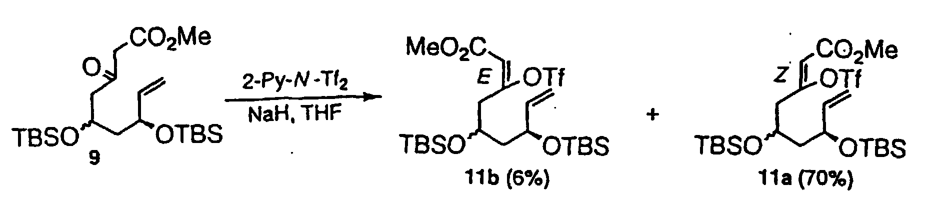

The methyl(5R and S,7S)-5,7-di(tert-butyldimethylsilyl)oxy-3-oxo-8-nonenoate (393.1 mg, 0.885 mmol) obtained in Example 8 was dissolved in THF (4.7 ml). To the solution, sodium hydride (60%; 41.1 mg, 1.03 mmol) was added under argon atmosphere at -5°C, followed by stirring for 1 hour. To the mixture, 2-[N,N-bis(trifuloromethylsulfonyl)amino]pyridine (333.2 mg, 1.05 mmol) was added, followed by further stirring at room temperature for 19.5 hours. The reaction mixture was mixed with water (1 ml) at 0°C, diluted with diethyl ether (80 ml) and washed with water (80 ml), a 10% aqueous citric acid solution (80 ml), 10% NaOH (80 ml) and a saturated aqueous sodium chloride (80 ml), in that order. The organic layer was dried over MgSO₄, filtered, concentrated and then purified by flash silica gel column chromatography (SiO₂ = 40 g, hexane:ethyl acetate = 50:1) to give the titled Compound 11a (320.7 mg, 0.557 mmol, 63%) as a colorless oil and the titled Compound 11b (27.9 mg, 0.0484 mmol, 6%) as a colorless oil. Compound 11a and Compound 11b were each obtained as a mixture of C-5 epimers (1:1). In addition, the staring Compound 9 (71.6 mg, 0.161 mmol, 18%) was recovered.
Compound 11a; ¹H NMR (300 MHz, CDCl₃) [0.01 (s), 0.03 (s), 0.05 (s), 0.07 (s), 12H], [0.086 (s), 0.088 (s), 0.89 (s), 18H], 1.62-1.88 (m, 2H), [2.47 (dd, J=8.1, 15.3 Hz), 2.53 (dd, J=5.3, 15.3 Hz), 1H], 2.63 (dd, J=5.1, 15.3 Hz, 0.5H], 2.75 (dd, J=4.2, 15.3 Hz, 0.5 Hz), [3.75 (s), 3.76 (s), 1H], 4.06 (quint., J=3.3 Hz, 1H), 4.15 (q, J=6.3 Hz, 0.5H), 4.27 (q, J= 4.8 Hz, 0.5H), [5.09 (br d, J=10.5 Hz), 5.14 (bd d, J=16.8 Hz), 5.17 (br d, J= 16.8 Hz), 2H)], [5.70-5.84 (m), 5.81 (s), 2H]
Compound 11b; ¹H NMR (300 MHz, CDCl₃) [0.016 (s), 0.022 (s), 0.034 (s), 0.051 (s), 0.055 (s), 0.062 (s), 0.069 (s), 12H], 0.085 (s, 9H), [0.87 (s), 0.89 (s), 9H], 1.62-1.89 (m, 2H), 2.96 (dd, J=5.4, 14.4 Hz, 0.5H), 3.09 (dd, J=5.4, 14.4 Hz, 0.5H), [3.26 (dd, J=6.0, 14.4 Hz), 3.28 (dd, J=6.0, 14.4 Hz), 1 Hz], [3.74 (s), 3.75 (s), 3H], [5.07 (br d, J=10.2 Hz), 1H], 5.78 (ddd, J=5.4, 10.2, 16.8 Hz, 1H), [5.98 (s), 5.99 (s), 1H]

### Example 10: Preparation of methyl(Z,3S-,5R and S)-[3,5-bis(tert-butyldimethylsilyl)oxy-2-methylenecyclohexylidene]acetate (Compound 12)

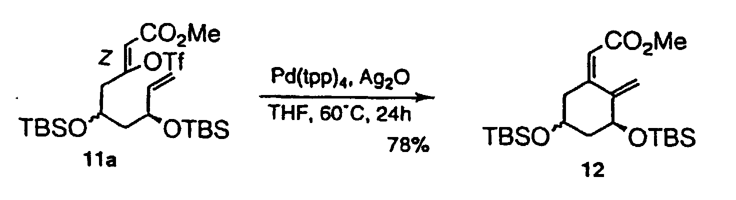

Under argon atmosphere, the methyl(Z,5R and S,7S)-5,7-bis(tert-butyldimethylsilyl)oxy-3-(trifluoromethanesulfonyl)oxynona-2,8-dienoate (355.5 mg, 0.617 mmol) obtained in Example 9, Ag₂O (157.6 mg, 0.676 mmol) and tetrakistriphenyiphosphine palladium (72.3 mg, 0.0626 mmol) were suspended in THF (35 ml) and stirred for 24 hours at 60°C. The reaction mixture was filtered through a silica gel column (SiO₂ = 8 g); the filtrate was concentrated and then purified by flash silica gel column chromatography (SiO₂ = 38 g, hexane:ethyl acetate = 50:1) to give a colorless oil of the titled Compound 12 (206.0 mg, 0.483 mmol, 78%) as a mixture of C-5 epimers (1:1). The epimer mixture was used in the next reaction step without further purification, however, a portion thereof was purified by preparative thin-layer silica gel chromatography for collecting data.
Compound 5R-12;¹H NMR (300 MHz) 0.06 (s, 6H), 0.09 (s, 3H), 0.10 (s, 3H), 0.87 (s, 9H), 0.94 (s, 9H), 1.57 (q, J=11.7 Hz, 1H), 2.15-2.27 (m, 2H), 2.38-2.50 (m, 1H), 3.63 (s, 3H), 3.64-3.80 (m, 1H), 4.06-4.12 (m, 1H), 4.96 (t, J=2.1 Hz, 1H), 5.25 (t, J=2.1 Hz, 1H), 5.67 (d, J=2.1 Hz, 1H);
FT-IR (neet) 1729, 1643, 1255, 1079, 874, 835, 776 cm⁻¹;
MS(EI) m/z 369 (100), 411, 426 (M⁺);
Compound 5S-12; ¹H NMR (300 MHz) 0.05 (s, 6H), 0.09 (s, 6H), 0.89 (s, 9H), 0.90 (s, 9H), 1.76 (ddd, J=3.0, 9.0, 12.1 Hz, 1H), 1.93 (dt, J=5.1, 12.1 Hz, 1H), 2.26 (dd, J=5.6, 13.1 Hz, 1H), 2.34 (br d, J=13.1 Hz, 1H), 3.63 (s, 3H), 4.24 (m, 1H), 4.53 (dd, J=4.2, 8.7 Hz, 1H), 4.98 (t, J=1.0 Hz, 1H), 5.18 (t, J=1.0 Hz, 1H), 5.63 (br s, 1H);
FT-IR (neet) 1722, 1641, 1253, 1077, 876, 834, 776 cm⁻¹;
MS(EI) m/z 369 (100), 411, 426 (M⁺);

### Example 11: Preparation of (Z,3S,5R)-2-[3,5-bis(tert-butyldimethylsilyl)oxy-2-methylenecyclohexylidene]ethanol (Compound 13) and (Z,3S,5S)-2-[3,5-bis(tert-butyldimethyl-silyl)oxy-2-methylenecyclohexylidene]ethanol (Compound 14)

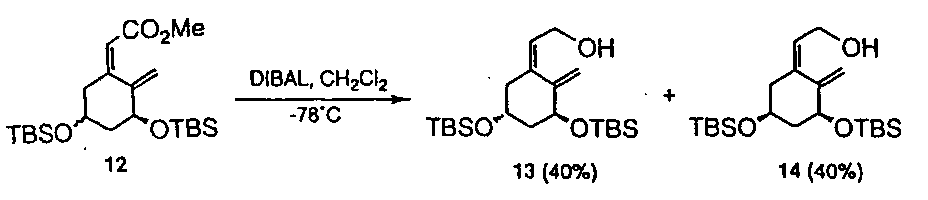

The methyl(Z,3S-,5R and S)-[3,5-bis(tert-butyldimethylsilyl)oxy-2-methylenecyclohexylidene]acetate (206.0 mg, 0.483 mmol) obtained in Example 10 was dissolved in CH₂Cl₂ (4.4 ml). Under argon atmosphere, the solution was cooled to -78°C; i-Bu₂AlH (as a 1.0M solution in toluene: 1.3 ml, 1.3 mmol) was slowly added dropwise to the solution, followed by stirring for 30 min. The reaction mixture was mixed with 3 droplets of a 10% aqueous sodium hydroxide solution, allowed to warm to room temperature while stirring and filtered with Celite; the filtrate was mixed with CH₂Cl₂ (40 ml) and a 10% aqueous citric acid solution (20 ml) for separation. The aqueous layer was further extracted with CH₂Cl₂ (20 ml x 2) and the organic layers were combined, dried over MgSO₄, filtered, concentrated and then purified by silica gel column chromatography (SiO₂ = 6 g, CH₂Cl₂) to give the known titled Compound 13 (76.6 mg, 0.192 mmol, 40%) as a colorless solid and the titled Compound 14 (77.1 mg, 0.194 mmol, 40%) as a colorless oil.

Compound 13 agreed with the standard in various spectrum data.
Compound 14; ¹H NMR (300 MHz) 0.06 (s, 6H), 0.07 (s, 3H), 0.08 (s, 3H), 0.89 (s, 9H), 0.93 (s, 9H), 1.56 (q, J=11.7 Hz, 1H), 2.15-2.21 (m, 2H), 2.42 (ddd, J=2.4, 5.1, 12.9 Hz, 1H), 3.73 (tt, J=2.1, 11.1 Hz, 1H), 4.14 (br d, J=12.6 Hz, 1H), 4.28 (dd, J=8.4, 12.6Hz, 1H), 4.76 (t, J=2.4 Hz, 1H), 5.33 (t, J=2.4 Hz, 1H), 5.55 (ddd, J=1.8, 5.1, 7.5 Hz, 1H);
¹³C NMR (75 MHz) -4.9, -4.6, 18.2, 18.5, 25.9, 46.4, 46.5, 60.0, 68.3, 70.1, 109.9, 126.9, 138.4, 147.4;
FT-IR (neat) 3353, 1467, 1254, 1080, 836 cm⁻¹;
MS(EI) m/z 73 (100), 341, 380, 398 (M⁺);
[α]_{D}²⁰ -65.6°(c 0.69, CHCl₃);
HRMS (EI) C₂₁H₄₂O₃Si₂ Calcd. 398.2672; Found 398.2647;
TLC (CH₂Cl₂, Rf 0.2; hexane : ethyl acetate = 4:1, Rf0.5)

### Example 12: Preparation of (Z,3S,5S)-2-[3,5-bis(tert-butyldimethylsilyl)oxy-2-methylenecyclohexylidene]-1-chloroethane (Compound 15)

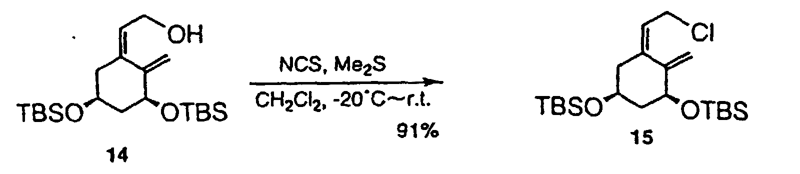

Under argon atmosphere, dimethyl sulfide (100 µl, 1.36 mmol) was added to a solution of N-chlorosuccinimide (181.0 mg, 1.36 mmol) in CH₂Cl₂ (4.4 ml) cooled at 0°C, followed by stirring for 40 min. At -20°C, 1.2 ml of the reaction mixture was slowly added dropwise via a syringe to a separately prepared solution of (Z,3S,5S)-2-[3,5-bis(tert-butyldimethylsilyl)oxy-2-methylenecyclohexylidene]ethanol (Compound 14) (74.1 mg, 0.186 mmol) in CH₂Cl₂ (0.6 ml). The mixture was stirred for 45 min. at -20°C and then the reaction temperature was slowly raised to room temperature over 30 min. The reaction mixture was mixed with hexane (20 ml); the organic layer was washed with water (20 ml) and saturated aqueous sodium chloride (20 ml), dried over MgSO₄, filtered and concentrated to give the titled Compound 15 (70.6 mg, 0.170 mmol, 91%) as a pale yellow oil.
¹H NMR (300 MHz) 0.07 (s, 6H), 0.08 (s, 3H), 0.09 (s, 3H), 0.89 (s, 9H), 0.93 (s, 9H), 1.56 (q, J=11.4 Hz, 1H), 2.18 (m, 2H), 2.43 (ddd, J=2.1, 4.8, 12.9 Hz, 1H), 3.71 (tt, J=4.5, 11.1 Hz, 1H), 3.94 (ddt, J=2.1, 4.5, 11.7 Hz, 1H), 4.10 (dd, J=1.5, 11.1 Hz, 1H), 4.18 (dd, J=8.1, 11.1 Hz, 1H), 4.96 (t, J=2.1 Hz, 1H), 5.39 (t, J=2.1 Hz, 1H), 5.57 (dt, J=2.1, 8.1 Hz, 1H);
¹³C NMR (75 MHz) -4.8, -4.6, 18.2, 18.5, 25.9, 41.5, 46.4, 46.5, 68.1. 70.0, 110.1, 123.2, 141.1, 146.9;
FT-IR (neat) 1467, 1377, 1255, 1078, 835 cm⁻¹;
MS(EI) m/z 57, 73 (100), 381, 401, 416 (M⁺);
HRMS (EI) C₂₀H₃₈O₂Si₂Cl Calcd. 401.2099; Found 401.2065;
TLC (hexane : ethyl acetate = 20:1, Rf0.5)

### Example 13: Preparation of (Z,3S,5S)-2-[[3,5-bis(tert-butyldimethylsilyl)oxy-2-methylenecyclohexylidene]ethyl]diphenylphosphineoxide (Compound 16)

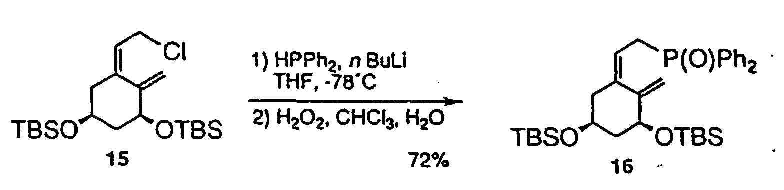

Under argon atmosphere, a solution of diphenylphosphine (135.5 mg, 0.728 mmol) in THF (2.4 ml) was cooled to 0°C, and n-BuLi (a 1.58M solution in hexane; 460 µl, 0.727 mmol) was slowly added dropwise to the solution and stirred for 10 min. Then the mixture was cooled to -50°C; the (Z,3S,5S)-2-[3,5-bis(tert-butyldimethylsilyl)oxy-2-methylenecyclohexylidene]-1-chloroethane (67.5 mg, 0.162 mmol) obtained in Example 12 was dissolved in THF (1 ml) and the solution was added dropwise to the mixture, followed by stirring for further 10 min. The reaction mixture was mixed with three droplets of water, allowed to warm to room temperature while stirring, and then mixed with CHCl₃ (7.4 ml) and 5% H₂O₂ (5.4 ml), followed by stirring for 1.5 hours. The reaction mixture was mixed with CHCl₃ (15 ml) for separation; the organic layer was washed with a saturated aqueous NaHSO₃ solution (20 ml) and water (20 ml), dried over MgSO₄, filtered, concentrated and then purified by silica gel column chromatography (SiO₂ = 25 g, hexane:ethyl acetate = 2:1) to give the titled Compound 16 (68.0 mg, 0.117 mmol, 72%) as a colorless viscous oil.
¹H NMR (500 MHz) 0.03 (s, 6H), 0.04 (s, 3H), 0.05 (s, 3H), 0.86 (s, 9H), 0.92 (s, 9H), 1.45 (q, J=11.5 Hz, 1H), 2.05-2.15 (m, 2H), 2.37 (ddd, J=2.0, 4.5, 12.0 Hz, 1H), 3.15 (dddd, J=2.5, 5.5, 15.0, 18.0 Hz, 1H), 3.32 (br dt, J=10.0, 15.0 Hz, 1H), 3.40 (tt, J=4.0, 11.5 Hz, 1H), 3.53 (ddt, J=2.0, 5.0, 9.0 Hz), 4.76 (t, J=2.5 Hz, 1H), 5.28 (t, J=2.5 Hz, 1H), 5.49 (ddt, J=2.0, 5.0, 7.5 Hz), 7.42-7.76 (m, 6H), 7.64-7.76 (m, 4H);
¹³C NMR -4.8, -4.7, -4.6, -4.5, 25.8, 25.9, 31.1, 32.2, 46.6, 46.8, 68.3, 69.22, 109.7, 115.7;
FT-IR (neat) 1471, 1437, 1253, 1120, 1073, 835 cm⁻¹;
MS(EI) m/z 525 (100), 582 (M⁺);
[α]_{D}²² -38.0°(c 1.02, CHCl₃);
HRMS (EI) C₃₃H₅₁O₃Si₂P Calcd. 582.3115; Found 582.3088;
TLC (hexane : ethyl acetate = 1:1, Rf0.5)

### Example 14: Preparation, of octahydro-1-[1'-(3''-methyl-3''-trimethylsilyloxy)ethyl]-7α-methylidene-4-on (ketonic compound 19)

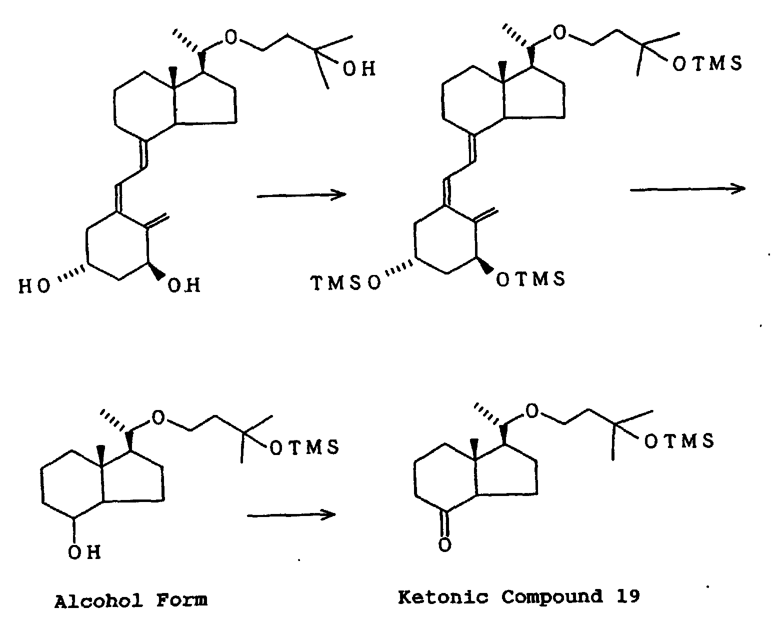

For preparing ketonic Compound 19, (5Z,7E)-(1S,3R,20S)-20-(3-hydroxy-3-methylbutyloxy)-9,10-secopregna-5,7,10(19)-triene-1,2,-diol (referred to as OCT in the specification), a known vitamin D derivative, was trimethylsilylated to give (5Z,7E)-(1S,3R,20S)-1,3-bis(trimethylsilyloxy)-20-(3-methyl-3-trimethylsilyloxy-butyloxy)-9,10-secopregna-5,7,10(19)-triene (referred to as OCT-tri(trimethylsilyl)ether in the specification), which was then subjected to ozonolysis and NaBH₄-reduction to give octahydro-1-[1'-(3''-methyl-3''-trimethylsilyloxy)ethyl]-7α-methylidene-4-ol (alcohol form). The alcohol form was oxidized with TPAP(n-Pr₄NRuO₄) to synthesize octahydro-1-[1'-(3''-methyl-3''-trimethylsilyloxy)ethyl]-7α-methylidene-4-one (ketonic compound 19), the CD ring structure of OCT.

### (1) Preparation of OCT-tri(trimethylsilyl)ether

To a solution of OCT (130.8 mg, 0.313 mmol) and triethylamine (0.36 ml, 1.583 mmol) in dichloromethane (5 ml), trimethylsilyloxy Tf (0.25 ml, 1.293 mmol) was added under a stream of argon while cooling with ice. After stirring for 1 hour, the reaction solution was diluted with diethyl ether, washed with water, dried over MgSO₄ and distilled under reduced pressure to remove the solvent. The residue was purified by column chromatography (SiO₂ = 10 g, hexane:ethyl acetate = 10:1) to give OCT-tri(trimethylsilyl)ether (186.3 mg, 94%) as a colorless solid.
[α]_{D}²⁶ +14.9 (c 0.37, CHCl₃);
IR (neat) 1251, 1075, 839 cm⁻¹;
¹H NMR (300 MHz, CDCl₃) d 6.27 (d, 1H, J=11.1 Hz), 6.05 (d, 1H, J=11.1 Hz), 5.20 (d, 1H, J=1.2 Hz), 4.90 (d, 1H, J=2.4 Hz), 4.37 (dd, 1H, J=6.6, 3.9 Hz), 4.18 (m, 1H), 3.65 (dt, 1H, J=8.7, 6.6 Hz), 3.32 (dt, 1H, J=8.7, 6.2 Hz), 3.20 (br quint, 1H, J=6.0 Hz), 2.84 (br d, 1H, J=12.3 Hz), 2.46 (dd, 1H, J=13.8, 3.8 Hz), 2.23 (dd, 1H, J=13.8, 7.8 Hz), 2.10-1.20 (m, 15H), 1.23 (s, 6H), 1.15 (d, 3H, J=6.0 Hz), 0.53 (s, 3H), 0.12, 0.10 (s x 2, 27H);
¹³C NMR (75 MHz, CDCl₃) d 148.0, 141.0, 135.0, 123.4, 118.1, 111.6, 78.1, 73.1, 71.7, 67.3, 65.0, 57.5, 56.3, 45.9, 44.9, 44.7, 44.6, 39.8, 30.5, 30.0, 25.8, 23.4, 22.2, 19.4, 12.6, 2.66, 0.31, 0.25

### (2) Preparation of alcohol form

The OCT-tri(trimethylsilyl)ether (185 mg, 0.292 mmol) obtained in the above (1) and NaHCO₃ (100 mg, 1.190 mmol) were dissolved in a mixture of dichloromethane (4 ml) and methanol (1 ml), and O₃ was aerated at -78°C for 30 min. To remove O₃, O₂ was aerated at -78°C for 10 min. and then methanol (5 ml) and NaBH₄ (200 mg, 5.263 mmol) were added to the mixed solution. After removing the ice bath, the reaction solution was stirred until the temperature thereof rose to 0°C. Almost all of the solvent was distilled off from the reaction solution under reduced pressure; the residue was mixed with water and extracted with dichloromethane. The extract was dried over MgSO₄ and concentrated under reduced pressure; the residue was purified by column chromatography (SiO₂ = 10 g, hexane:ethyl acetate = 8:1) to give the alcohol form (74.3 mg. 72%) as a colorless oil.
[α]_{D}²⁵ +38.4 (c 0.37, CHCl₃);
IR (neat) 3455, 1250, 1169, 1036, 842 cm⁻¹;
¹H NMR (300 MHz, CDCl₃) d 4.08 (br q, 1H, J=2.4 Hz), 3.64 (ddd, 1H, J=9.0, 8.1, 6.3 Hz), 3.32 (ddd, 1H, J=9.0, 8.1, 6.6 Hz), 3.18 (dq, 1H, J=8.7, 6.3 Hz), 2.14-1.25 (m, 13H), 1.22 (s, 6H), 1.13 (d, 3H, J=6.3 Hz), 0.91 (s, 3H), 0.09 (s, 9H),
¹³C NMR (75 MHz, CDCl₃) d 77.9, 73.2, 69.3, 64.9, 57.6, 52.6, 44.6, 41.0, 39.7, 33.6, 30.5, 25.7, 22.5, 19.2, 17.4, 14.2, 2.66

### (3) Preparation of ketonic form

The alcohol form (22.2 mg, 0.062 mmol) obtained in the above (2) was dissolved in dichloromethane (4 ml). To the solution, N-methylmorpholine N-oxide (NMO) (11.4 mg, 0.097 mmol) and 4A molecular sieves (21.8 mg) were added, followed by stirring under a flow of argon at room temperature, and then mixed with n-Pr₄NRuO₄ (1.0 mg, 0.0028 mmol). After 5 hours, the reaction solution was filtered with Celite; the filtrate was concentrated under reduced pressure. The residue was purified by column chromatography (SiO₂ = 1 g, hexane:ethyl acetate = 5:1) to give octahydro-1-[1'-(3''-methyl-3''-trimethylsilyloxy)ethyl]-7α-methylidene-4-one (ketonic compound 19) (20.2 mg, 93%) as a colorless oil.
IR (neat) 1716, 1251, 1036, 839 cm⁻¹;
¹H NMR (300 MHz, CDCl₃) d 3.65 (ddd, 1H, J=9.3, 8.1, 6.6 Hz), 3.31 (ddd, 1H, J=9.3, 8.7, 6.6 Hz), 3.22 (dq, 1H, J=7.5, 6.4 Hz), 2.44 (dd, 1H, J=11.4, 7.5 Hz), 2.25 (m, 2H), 2.10-1.48 (m, 11H), 1.22 (s, 6H), 1.17 (d, 3H, J=6.0 Hz), 0.62 (s, 3H), 0.09 (s, 9H),
¹³C NMR (75 MHz, CDCl₃) d 211.9, 65.2, 61.9, 57.5, 48.9, 44.5, 41.0, 38.2, 30.5, 30.4, 25.2, 23.9, 19.3, 19.1, 13.3, 2.68

### Example 15: Preparation of (5Z,7E)-(1S,3S,20S)-1,3-bis(tert-butyldimethylsilyloxy)-20-(3-methyl-3-trimethylsilyloxybutyloxy)-9,10-secopregna-5,7,10(19)-triene (Compound 20)

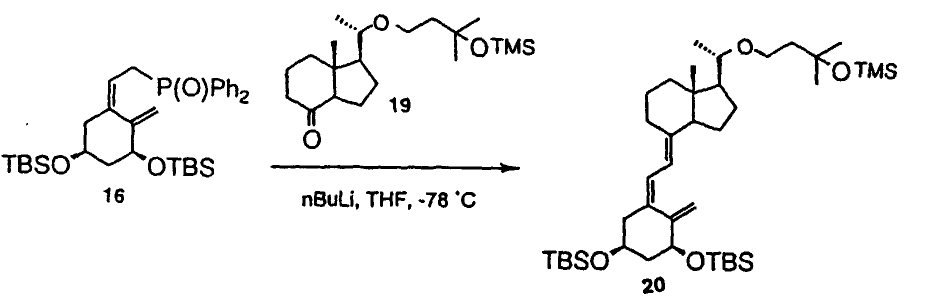

The (Z,3S,5S)-2-[[3,5-bis(tert-butyldimethylsilyl)oxy-2-methylenecyclohexylidene]ethyl]diphenylphosphineoxide (Compound 16) (43.3 mg, 0.0745 mmol) obtained in Example 13 was dissolved in THF (0.7 ml). To the solution, n-BuLi (a 1.58M hexane solution; 47 µl, 0.0745 mmol) was added under argon atmosphere at -78°C and the mixture was stirred for 8 min. Thereafter, the octaydro-1-(1'-(3''-methyl-3''-trimethylsilyloxy)ethyl]-7α-methyliden-4-one (ketonic compound 19) (13.1 mg, 0.0370 mmol) obtained in Example 14 was dissolved in THF (2 ml), and the solution was slowly added dropwise to the mixture followed by stirring for 6 hours. The reaction mixture was mixed with aqueous NH₄Cl (5 ml), allowed to warm to room temperature, mixed with CH₂Cl₂ (10 ml) and water (1 ml) for separation; then the aqueous layer was extracted with CH₂Cl₂ (10 ml x 2). The organic layers were combined, dried over Na₂SO₄, filtered, concentrated and then purified by preparative thin-layer chromatography (0.5 mm x 1, hexane:ethyl acetate = 4:1) to give the titled Compound 20 (12.6 mg, 0.0165 mmol, 45%) as a colorless oil.
¹H NMR (300 MHz, CDCl₃) 0.07 (s, 6H), 0.08 (s, 3H), 0.10 (s, 12H), 0.54 (s, 3H), 0.89 (s, 9H), 0.94 (s, 9H), 1.15 (d, J=6.3 Hz, 3H), 1.23 (s, 6H), 1.42-1.76 (m, 12H), 1.84-2.04 (m, 3H), 2.16-2.26 (2H), 2.43 (br dd, J=2.7, 12.3 Hz, 1H), 2.83 (br d, J=11.1 Hz, 1H), 3.21 (quint., 7=6.6 Hz, 1H), 3.12 (q, J=6.6 Hz, 1H), 3.65 (q, J=6.6 Hz, 1H), 3.65-3.76 (m, 1H), 3.95 (br d, J=11.1 Hz, 1H), 4.93 (t, J=2.4 Hz, 1H), 5.38 (t, J=2.4 Hz, 1H), 6.01 (d, J=11.4 Hz, 1H), 6.27 (d, J=11.4 Hz, 1H);
¹³C NMR (75 MHz, CDCl₃) -5.0, -4.9, -4.6, 2.7, 12.5, 18.3, 18.6, 19.4, 22.2, 23.5, 25.7, 26.0, 29.1, 29.8, 30.5, 39.7, 44.6, 44.8, 46.6. 47.0, 56.3, 57.5, 65.0, 68.8, 70.3, 78.0, 110.0, 117.9, 123.0, 134.9, 142.0, 148.1;
FT-IR (neat) 1644, 1471, 1370, 1250, 1075, 909, 875, 835 cm⁻¹;
MS(EI) m/z 75 (100), 703, 718 (M⁺);
HRMS (EI) C₄₁H₇₈O₄Si₃ Calcd. 718.5208; Found 718.5201;
TLC (hexane : ethyl acetate = 20:1, Rf0.3)

### Example 16: Preparation of (5Z,7E)-(1S,3S,20S)-20-(3-hydroxy-3-methylbutyloxy)-9,10-secopregna-5,7,10(19)-triene-1,3-diol (Compound 21)

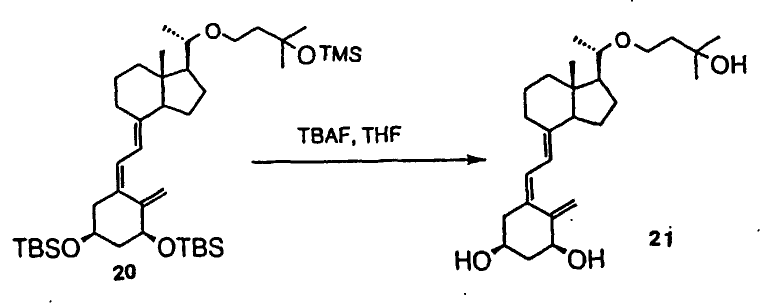

The (5Z,7E)-(1S,3S,20S)-1,3-bis(tert-butyldimethylsilyloxy)-20-(3-methyl-3-trimethylsilyloxybutyloxy)-9,10- secopregna-5,7,10(19)-triene (Compound 20) (12.6 mg, 0.0165 mmol) obtained in Example 15 was dissolved in THF (0.3 ml). To the solution, a solution of n-tetrabutylammonium fluoride (a 1.0M solution in THF; 70 µl, 0.070 mmol) was added under argon atmosphere, followed by stirring for 18 hours. The reaction mixture was mixed with water (3 ml) and extracted with ethyl acetate (5 ml x 3); the extracts were dried over Na₂SO₄, filtered, concentrated and then purified by preparative thin-layer chromatography (0.5 mm x 1, hexane:ethyl acetate = 1:15) to give the titled Compound 21 (7.7 mg, 0.0184 mmol, quant.) as a colorless oil.
¹H NMR (300 MHz, CDCl₃) 0.53 (s, 3H), 1.19 (d, J=6.0 Hz, 3H), 1.24 (s, 6H), [1.44-1.74 (m), 1.75 (t, J=5.7 Hz), 12H], 1.84-2.06 (m, 2H), 2.25 (br s, 1H), 2.44 (dd, J=5.1, 13.2 Hz, 1H), 2.56 (br dd, J=2.7, 13.2 Hz, 1H), 3.25 (quint., J=6.3 Hz, 1H), 3.48 (dt. J=6.0, 9.3 Hz. 1H), 3.82 (br s, 1H), 3.85 (dt, J=5.7, 9.3 Hz, 1H), 4.06 (br q, J=5.1 Hz, 1H), 4.32 (br q, J=3.9 Hz, 1H), 5.25 (d, J=1.8 Hz, 1H), 5.55 (br s, 1H), 6.28 (d, J=11.1 Hz, 1H), 6.68 (d, J=11.1 Hz, 1H);
¹³C NMR (75 MHz, CDCl₃) 12.8, 18.9, 22.3, 23.3, 25.7, 29.0, 29.2, 29.4, 39.7, 40.7, 41.6, 44.9, 45.6, 56.2, 57.2, 65.6, 68.3, 70.6, 73.3, 78.9, 113.1, 117.5, 125.5, 132.1, 142.4, 147.2;
FT-IR (neat) 3319, 1453, 1371, 1077, 898 cm⁻¹;
MS(EI) m/z 44 (100), 400, 418 (M⁺);
[α]_{D}¹⁷ -33.0°(c 0.39, EtOH);
HRMS (EI) C₂₆H₄₂O₄ Calcd. 418.3083; Found 418.3070;
TLC (hexane : ethyl acetate = 1:10, Rf0.4)

### INDUSTRIAL APPLICABILITY

Recently, it is reported that 1α,25-dihydroxy vitamin D₃ is metabolized in vivo to 3-epi-1α,25-dihydroxy vitamin D₃ (Tenth Workshop on Vitamin D, Strasbourg, France, May 24-29, 1997, Abstract, p. 246). Therefore, one may predict that the final end compounds of the present invention, 1α,3α-dihydroxy-22-oxavitamin D derivatives having the general formula (I), can also be produced by metabolizing a 1α,3β-dihydroxy-22-oxavitamin D derivative which is known to have the ability to induce differentiation and inhibit proliferation *in vivo*.

Therefore, similarly to the 1α,3β-dihydroxy-22-oxavitamin D derivative, 1α,3α-dihydroxy-22-oxavitamin D derivatives of the present invention which have the general formula (I) are expected to have the ability to induce differentiation and inhibit proliferation. In other words, the present invention can provide novel vitamin D derivatives that possibly have useful physiological activities such as the ability to induce differentiation and inhibit proliferation.

Moreover, compounds having the general formula (III) and compounds having the general formula (IV) are important as intermediates for preparing the compounds having the general formula (I); by using these compounds of the general formula (III) and (IV), the final end compounds of the present invention which have the general formula (I) can be readily prepared.

## Claims

1. A 22-oxavitamin D derivative having the following general formula (I): 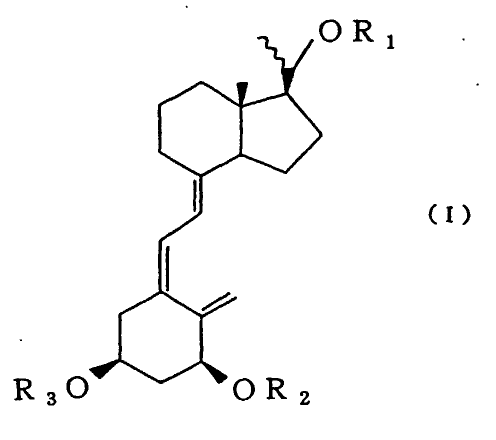 (wherein R₁ represents a straight or branched C₄-C₁₀ alkyl group having 1 to 3 hydroxy groups, and R₂ and R₃ each independently represents a hydrogen atom or a protecting group).

2. A 22-oxavitamin D derivative as set forth in claim 1, wherein R₁ represents a group having the following general formula (II): 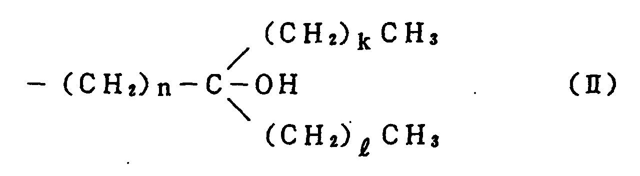 (wherein n represents an integer of 1-3 and k and l each independently represents an integer of 0-2).

3. A 22-oxavitamin D derivative as set forth in claim 1, wherein R₁ represents 3-hydroxy-3-methylbutyl group, a 2-hydroxy-3-methylbutyl group, a 4-hydroxy-3-methylbutyl group, a 2,3-dihydroxy-3-methylbutyl group, a 2,4-dihydroxy-3-methylbutyl group, a 3,4-dihydroxy-3-methylbutyl group, a 3-hydroxy-3-ethylpentyl group, a 2-hydroxy-3-ethylpentyl group, a 4-hydroxy-3-ethylpentyl group, a 2,3-dihydroxy-3-ethylpentyl group, a 2,4-dihydroxy-3-ethylpentyl group, a 3,4-dihydroxy-3-ethylpentyl group, a 4-hydroxy-4-methylpentyl group, a 3-hydroxy-4-methylpentyl group, a 5-hydroxy-4-methylpentyl group, a 3,4-dihydroxy-4-methylpentyl group, a 3,5-dihydroxy-4-methylpentyl group, a 4,5-dihydroxy-4-methylpentyl group, a 3-hydroxy-3-(n-propyl)hexyl group, a 4-hydroxy-3-(n-propyl)hexyl group, a 2-hydroxy-3-(n-propyl)hexyl group, a 23-dihydroxy-3-(n-propyl)hexyl group, a 3,4-dihydroxy-3-(n-propyl)hexyl group, a 2,4-dihydroxy-3-(n-propyl)hexyl group, a 3-hydroxy-4-ethylhexyl group, a 4-hydroxy-4-ethylhexyl group, a 5-hydroxy-4-ethylhexyl group, a 3,4-dihydroxy-4-ethylhexyl group, a 3,5-dihydroxy-4-ethylhexyl group, a 4,5-dihydroxy-4-ethylhexyl group, a 4-hydroxy-5-methylhexyl group, a 5-hydroxy-5-methylhexyl group, a 6-hydroxy-5-methylhexyl group, a 4,5-dihydroxy-5-methylhexyl group, a 4,6-dihydroxy-5-methylhexyl group, a 5,6-dihydroxy-5-methylhexyl group, a 5-hydroxy-6-methylheptyl group, a 6-hydroxy-6-methylheptyl group, a 7-hydroxy-6-methylheptyl group, a 5,6-dihydroxy-6-methylheptyl group, a 5,7-dihydroxy-6-methylheptyl group, a 6,7-dihydroxy-6-methylheptyl group, a 4-hydroxy-5-ethylheptyl group, a 5-hydroxy-5-ethylheptyl group, a 6-hydroxy-5-ethylheptyl group, a 4,5-dihydroxy-5-ethylheptyl group, a 4,6-dihydroxy-5-ethylheptyl, a 5,6-dihydroxy-5-ethylheptyl group, a 3-hydroxy-4-(n-propyl)heptyl group, a 4-hydroxy-4-(n-propyl)heptyl group, a 5-hydroxy-4-(n-propyl)heptyl group, a 3,4-dihydroxy-4-(n-propyl)heptyl group, a 3,5-dihydroxy-4-(n-propyl)heptyl group or a 4,5-dihydroxy-4-(n-propyl)heptyl group.

4. A 22-oxavitamin D derivative as set forth in claim 1, wherein R₁ represents a 3-hydroxy-3-methylbutyl group.

5. A 22-oxavitamin D derivative as set forth in claim 1, wherein the 20-position thereof is in S-configuration.

6. A 22-oxavitamin D derivative as set forth in claim 2, wherein the 20-position thereof is in S-configuration.

7. A 22-oxavitamin D derivative as set forth in claim 3, wherein the 20-position thereof is in S-configuration.

8. A 22-oxavitamin D derivative as set forth in claim 4, wherein the 20-position thereof is in S-configuration.

9. A compound having the general formula (III): 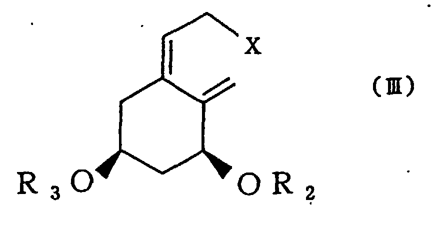 (wherein X represents a hydroxy group, a chlorine atom or a diphenylphosphine oxide group, and R₂ and R₃ each independently represents a hydrogen atom or a protecting group).

10. A compound having the general formula (IV): 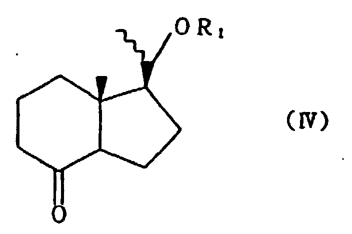 (wherein R₁ represents a straight or branched C₄-C₁₀ alkyl group having 1 to 3 hydrogen groups).

11. A method of producing a 22-oxavitamin D derivative having the following general formula (I): 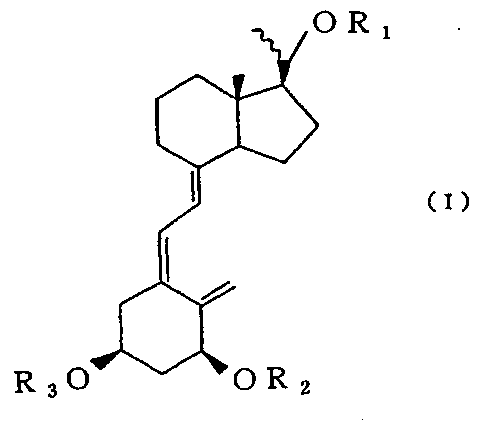 (wherein R₁ represents a straight or branched C₄-C₁₀ alkyl group having 1 to 3 hydroxy groups, and R₂ and R₃ each independently represents a hydrogen atom or a protecting group),
said method comprising reacting a compound having the following general formula (IIIa): 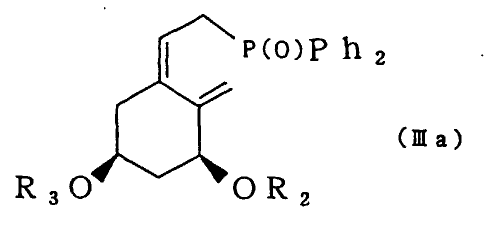 (wherein R₂ and R₃ each independently represents a hydrogen atom or a protecting group)
with a compound having the following general formula (IV): 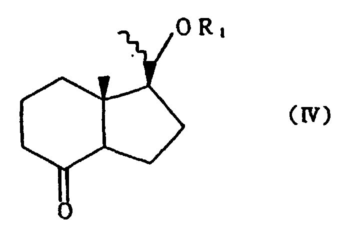 (wherein R₁ represents a straight or branched C₄-C₁₀ alkyl group having 1 to 3 hydroxy groups)
in a solvent in the presence of a base.
